Europäisches Patentamt

European Patent Office

Office européen des brevets

(11) Publication number: **0 124 212**
**B1**

(12) **EUROPEAN PATENT SPECIFICATION**

(45) Date of publication of patent specification: **26.11.86**

(51) Int. Cl.⁴: **C 12 M 1/22, B 65 D 21/02**

(21) Application number: **84301148.7**

(22) Date of filing: **22.02.84**

(54) **Petri dish.**

(30) Priority: **02.05.83 US 490271**

(43) Date of publication of application:
**07.11.84 Bulletin 84/45**

(45) Publication of the grant of the patent:
**26.11.86 Bulletin 86/48**

(84) Designated Contracting States:
**BE DE FR GB IT NL SE**

(56) References cited:
**FR-A-1 284 822**
**FR-A-1 572 527**
**FR-A-1 601 975**
**FR-A-2 486 915**
**GB-A-2 035 277**

(73) Proprietor: **Becton, Dickinson and Company**
**Mack Centre Drive P.O. Box 2224**
**Paramus New Jersey 07652-1149 (US)**

(72) Inventor: **Ervin, Klon Randol**
**4101 Morgan Mill Road**
**Glen Arm Maryland 21057 (US)**

(74) Representative: **Ruffles, Graham Keith et al**
**MARKS & CLERK 57-60 Lincoln's Inn Fields**
**London WC2A 3LS (GB)**

Courier Press, Leamington Spa, England.

**Description**

Background of the invention
1. Field of the invention

The present invention relates to an improved microorganism culturing dish, commonly referred to as a Petri dish. More particularly, the present invention is directed to a Petri dish with improved properties in respect to the ability to arrange one or more similar dishes to form a relatively stable stack of dishes, that is so structured and dimensioned as to enable a medical technician using the Petri dish to utilize the dish with increased efficiency and comfort, and has features which permit more efficient use of the media required to culture microorganisms.

Petri dishes generally consist of a bottom container for the medium which has a bottom wall and a single upstanding, cylindrical, peripheral side wall. The container is used in combination with a slightly larger lid having a top wall which merges into a downwardly extending cylindrical peripheral side wall. The lid telescopically covers the container.

While the Petri dish is a seemingly simple product, it must meet many conflicting requirements of the manufacturer and the user. It is not uncommon that as many as a thousand Petri dishes are used by a single technician in a single day. Efficient operations dictate that the technician must not have to use both hands to remove or replace the lid. Also it is common practice to work from an inverted stack of Petri dishes wherein the container is picked up with one hand, leaving the other hand free to streak a sample across a solidified media located in the bottom portion of the container.

It is also common practice for the laboratory technician to transport a stack of Petri dishes which have been inoculated from one portion of the laboratory to another. Accordingly, it is usual to provide some type of stacking restraint which prohibits sliding of the Petri dishes while they are being transported in a stack. A common practice is to provide a circumferential rib on the bottom of the container which mates with a circumferential rib of slightly larger or smaller diameter on the top of the lid of the Petri dish which is below the container in a stack. These mating ribs act to restrain movement of the Petri dish and prevent the stack from collapsing while it is being transported. Such arrangement of circumferential ribs has not been wholly satisfactory and has not completely prevented the problem of sliding while the stack of Petri dishes is carried.

A further problem is that, when solidified media is prepackaged in the bottom container, the solidified media tends to separate from the area adjacent to the junction of the side wall and the bottom wall of the bottom container during shipping and storage.

2. Prior art

United States Patent No. 4,160,700 to Boomus et al describes a Petri dish having an outwardly extending radial flange in the lid of a Petri dish to permit easy removal of the lid. The Boomus et al patent also discloses the use of a mating arrangement of circumferential ribs on the lid and container sections of the Petri dish which engage to inhibit sliding of a stack of Petri dishes. United States Patent No. 3,198,713 to McCormick describes a Petri dish which is adapted to be arranged with one or more similar dishes to form a relatively stable stack of dishes. In the arrangement of the McCormick patent a plurality of open-top Petri dishes is arranged to form a stable stack wherein the base dish provides the lid for an underlying base dish. United States Patent No. 3,649,463 to Buterbaugh describes a Petri dish having a bottom portion containing an outwardly extending peripheral flange provided with a plurality of inwardly extending slots. The flange is spaced upward from the bottom portion and away from the lower edge of the top portion to permit grasping of the flange by the fingers.

3. Summary of the invention

The present invention is directed to an improved Petri dish having an overall construction which facilitates easy stacking and transport. The improved Petri dish of the invention provides an improved ease in manipulation either through grasping the top lid of the Petri dish or the bottom container of the Petri dish for use by the laboratory technician in applying a specimen or sample to a solidified media contained in the bottom portion of the container.

In general, the Petri dish of the present invention has a transition zone which comprises:

(a) a first straight section adjacent the side wall which extends from the side wall toward the bottom wall at an angle A relative to the plane of the bottom wall, the angle A preferably being from about 1° to about 5°, more preferably about 3°,

(b) a curved section adjacent the first straight section; and

(c) a second straight section adjacent the curved section which extends from the curved section to the bottom wall at an angle B relative to the plane of the bottom wall, the angle B being from about 80° to about 90°, preferably about 90°.

The configuration of the transition zone increases the total area of the bottom container in contact with the media but does not reduce the overall thickness and depth of the solidified media. The provision of the straight and curved sections at the various portions of the transition zone facilitates the formation of a meniscus on the media as it solidifies and at the same time reduces media fallout during storage, handling and shipping, once the gelling agent in the media has solidified.

The Petri dish of the present invention preferably also includes an indentation in the lid of the Petri dish to permit plates to be stacked and provide improved stability of the stack during transportation. The indentation in the lid suitably mates with a circumferential rib formed in the bottom wall of the container of the Petri dish.

More specifically, a projection can be provided on the bottom surface of the bottom wall, for instance by extending the second straight section beyond the junction of the straight section with the bottom wall to provide a circumferential projection conveniently of arcuate cross-section. The lid preferably has a substantially flat top wall merging into a downwardly extending substantially cylindrical side wall having an inner cylindrical surface with a diameter slightly larger than the diameter of the upwardly extending side wall of the container, the top wall merging through a straight slanted section to a depressed area which is radially spaced from the side wall a distance sufficient to receive the projection of a container when a plurality of Petri dishes are stacked. The straight slanted section then preferably leads from the top wall to the depressed area at an angle D relative to a line normal to the plane of the top wall of from about 10° to about 18°, preferably about 15°, and the termination of the inner surface of the side wall preferably has an arcuate projection.

In a typical Petri dish of this invention, the radius of curvature of the curved section is from about 0.5 to about 1.5 percent of the diameter of the bottom wall, preferably from about 0.7 to about 0.9 percent of the diameter of the bottom wall. The height of the container from the top of the side wall to the bottom of the bottom wall is suitably from about 10 to about 15 mm. The diameter at the top of the wall is preferably from about 75 to about 100 mm. The side wall preferably extends from the bottom wall at an angle C from a line normal to the bottom wall of from about 0° to about 5°, preferably about 3°. The outside cylindrical surface of the side wall can be abraded in an area extending upwardly from the junction of the side wall and transition zone. Preferably the side wall extends downwardly at an angle E normal to the plane of the top wall of from about 0° to about 5°, preferably about 3°.

4. Description of the drawings

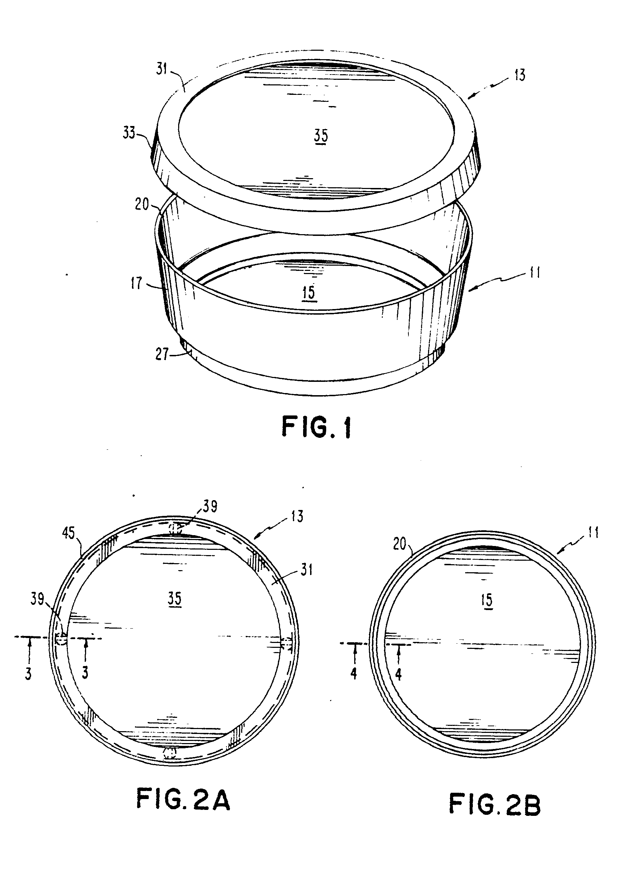

Figure 1 is a perspective view of the Petri dish of the invention showing a lid and a container.

Figure 2a is a top plan view of the lid of the Petri dish.

Figure 2b is a top plan view of the container of the Petri dish.

Figure 3 is an enlarged partial sectional view taken along line 3—3 of Figure 2a.

Figure 4 is an enlarged partial sectional view taken along line 4—4 of Figure 2b.

Figure 5a is a top plan view of the lid of a prior art Petri dish.

Figure 5b is a top plan view of a container of a prior art Petri dish.

Figure 6 is an enlarged partial sectional view taken through line 6—6 of Figure 5a.

Figure 7 is an enlarged partial sectional view taken along line 7—7 of Figure 5b.

Figure 8 shows the relationship of a stack of Petri dishes made in accordance with the present invention.

Figures 9a—9d are a sequence of schematic drawings illustrating an improved feature of the lid of the Petri dish of the invention which facilitates mating of the lid and the container of the Petri dish during manufacturing.

5. Detailed description of the invention

Generally for culturing microorganisms, two types of medium are in common use: liquid medium in which the microorganisms settle on the bottom surface of the culture dish, and solidified medium in which the microorganisms are dispersed throughout the solidified culture medium. Solidified cultured medium is usually solidified through the use of a hydrocolloid, such as agar. The present invention is directed to Petri dishes which use solidified medium which is deposited in a bottom container of the Petri dish.

Referring now to the drawings, the Petri dish of the invention consists of a container 11 and a lid 13 for the container. Both the container 11 and the lid 13 are of unitary molded construction and are formed from an organic polymer. At least the lid 13 should be transparent to enable inspection of the culture grown in the Petri dish and it is preferred that both the lid and the container be molded of a transparent polymer. Preferred polymers are polystyrene and polyparamethyl styrene. Other suitable polymers include polypropylene, polycarbonate, poly-vinylidene chloride, and styrene acrylonitrile.

The container has a flat, round bottom wall 15 from which there extends substantially perpendicularly upwardly a substantially cylindrical side wall 17. The side wall 17 merges into the bottom wall 15 through a transition zone. The enlarged upper section 20 of the side wall is provided for ease in releasing the molded container from the injection mold after the container is formed and cooperates with an arcuate portion of the lid 13 during automated assembly.

The transition zone has a first substantially straight section 21 and a substantially curved section 23. The first straight section merges into the side wall 17 and the radii of the junction of the straight section 21 and the side wall 17 is made as small as is consistent with injection molding practice. The first straight section 21 leads toward the bottom wall 15 at an angle A which is preferably from about 1 to about 5 degrees, preferably about 3°, in relation to the plane of the bottom wall 15. The curved section 23 has a radius of curvature R which is preferably from about .5 to about 1.5 percent of the diameter of the bottom wall 15, most preferably from about .7 to about .9 percent. The first straight section of the transition zone 19 merges through curved section 23 into a second straight section 25. The second straight section 25 is at an angle B, which is preferably substantially normal to the plane of the bottom wall 15, but may be from about 80° to about 90°. Angle B should not exceed about 92° to prevent interference during stacking of the container 11 and the lid 13.

The second straight section 25 extends downwardly from the bottom edge of the bottom wall 15 to provide a circumferential projection 27. This circumferential projection is concentric with the side wall 17 and is of less diameter than that of the side wall 17. The circumferential projection 27 is of arcuate cross section. The radius of the arc is not critical and is primarily provided for ease in molding of the container 11.

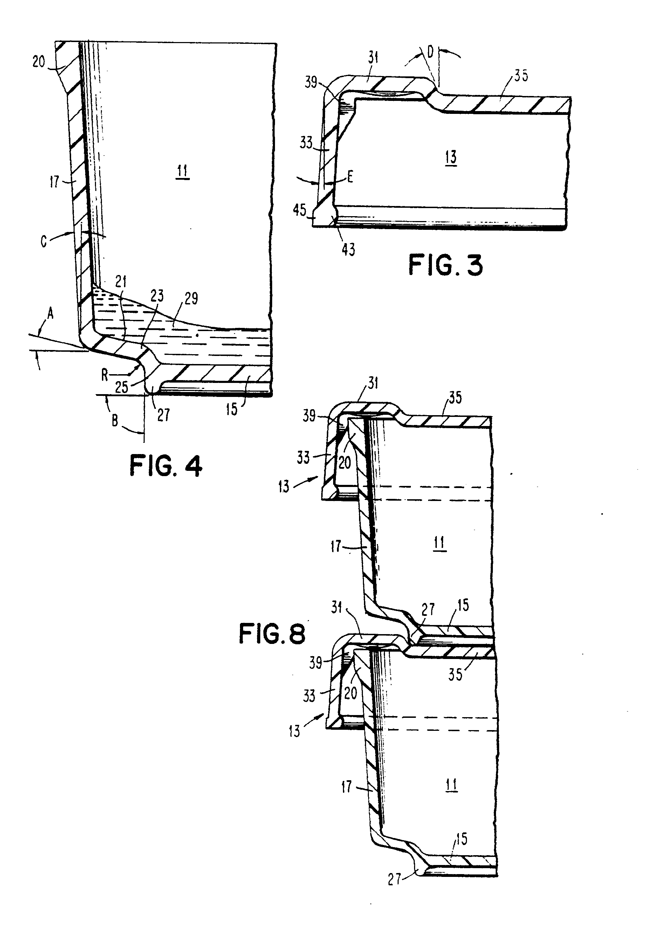

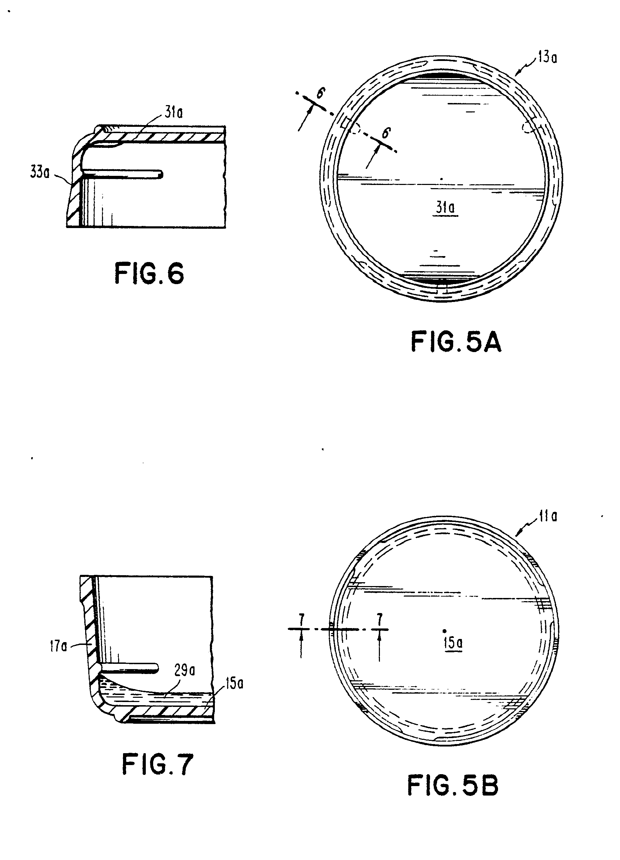

As shown in Figure 4, the medium 29 forms an upwardly curving meniscus. It has been demonstrated that the construction of the container of the Petri dish of the present invention, having the transition zone 19, results in better media holding than the prior art containers wherein the side wall extends substantially downward till it merges with the bottom wall, such as shown in Figure 7. Also, as shown in Figure 4, the media has a substantially constant depth through the transition zone which is the same as the depth of the media over the bottom wall 15. This results in a substantial alleviation of a "halo" effect.

The diameter of the container 11 at the top of side wall 17 is preferably from about 75 to about 100 millimeters. The total height of the container 11 from the top of the bottom wall 15 to the bottom of the circumferential projection 27 is preferably from about 10 to about 15 mm. Side wall 17 is usually inclined from a line normal to bottom wall 15 by an angle C for ease of removal from the mold. Angle C is preferably from about 0 to about 5 degrees, most preferably about 3°.

The portion of the bottom wall 15 immediately adjacent to the first straight section 21 of the transition zone is preferably treated with an abrasive grit during the molding process to provide a frosted surface. The provision of a frosted surface at this portion of the side wall serves to aid in grasping the container in one hand of the laboratory technician.

The lid 13 of the Petri dish has a flat, round top wall 31 which has extending substantially perpendicularly downwardly therefrom a substantially cylindrical side wall 33. The side wall 33 is usually inclined from a line normal to the top wall 31 by an angle E for ease of removal from the mold. Angle E is preferably from about 0 to about 5 degrees, most preferably about 3°. The depth of the side wall 33 is less than the height of side wall 17. Preferably the side wall 33 has a depth of from about .50 to about .60 percent of the depth of the container 11. For example, a preferred depth for the side wall 33 is 7.5 mm for a container depth of 13.33 mm.

The top wall 31 is provided with a depressed area 35. The edge 37 of the depressed area 37 is radially spaced from the side wall 33 a distance to receive the circumferential projection 27 of the container. This establishes a stackable feature which prevents the container from sliding away from the lid 13 when a stack of Petri dishes is prepared. The top wall 31 leads to the depressed area 35 through a straight edge section 37. The angle D that the edge section 37 makes with a line normal to the plane of the top wall 31 has been found to be important. This angle should be from about 10 to about 18 degrees, preferably about 15 degrees. Larger angles result in a failure of the combination of the depressed area 35 and the circumferential projection 27 to prevent sliding and a lessened angle results in reducing the ease of stability of the Petri dishes of the invention.

A stacking lug 39 is provided at spaced intervals around the inside of top wall 31. The stacking lug serves to reduce the spacing between the side wall 17 of the container and the side wall 33 of the lid to prevent undue movement of the lid when in place over the container. The stacking lug 17 is provided in accordance with known practice which is generally shown and described in United States Patent No. 4,321,330. The top portion 41 of the stacking lug 39 serves to raise the lid 13 from the container 11 when the lid 13 is in place. This provides a channel for air to pass between the lid and the container.

The side wall 33 terminates in an inner arcuate portion 43 and outer strengthening portion 45. The strengthening portion 45 provides additional bulk to assist in extracting the lid from the injection mold. The arcuate portion 43 is designed to assist in the automated assembly of the lid 13 with the container 11 by mechanical equipment.

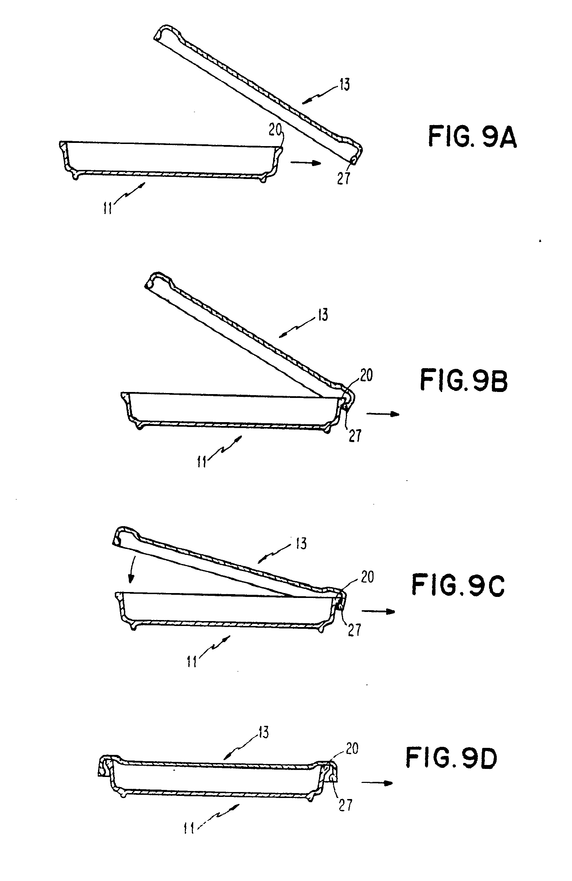

A sequence of steps is shown in Figure 9 which illustrates the use of the arcuate portion 43 of the lid 13 during automated assembly. The arcuate portion 43 provides a camming action to assist in locating lid 13 over the container 11 during such automated assembly through cooperation with the stiffened section.

Figure 8 shows the relationship of the circumferential projection 27 and the depressed area 35 when a series of Petri dishes are placed in a stacked array. As shown, the circumferential projection 27 abuts the bottom of the depressed area at the depressed area edge 37. The top of the side wall 17 rests against the top portion 41 of the stacking lug 39. It should be noted that the depressed area 35 needs only to be wide enough to receive the circumferential projection 27 and does not need to extend across the full area of top wall 31, as shown.

Figures 5, 6 and 7 illustrate a prior art Petri dish for comparative purposes. Elements of the prior art Petri dish which are common to the Petri dish of the invention are shown with common numbers and an "a" suffix.

The modifications made in the container lid of the Petri dish of the present invention provide a solution to numerous problems which have been present in the Petri dish industry for some time.

**Claims**

1. A Petri dish for the culturing of microorganisms comprising:
   a lid (13) and a bottom container (11) having a substantially flat bottom wall (15) merging into an upstanding, substantially cylindrical, side wall (17) through a transition zone extending between the bottom wall and the side wall; characterized in that the transition zone includes:

(a) a first straight section (21) adjacent the side wall which extends from the side wall toward the bottom wall at an angle A relative to the plane of the bottom wall,

(b) a curved section (23) adjacent the first straight section; and

(c) a second straight section (25) adjacent the curved section which extends from the curved section to the bottom wall at an angle B relative to the plane of the bottom wall.

2. A Petri dish in accordance with Claim 1 wherein the angle A is from about 1° to about 5°, preferably about 3°.

3. A Petri dish in accordance with Claim 1 or 2, wherein the angle B is from about 80° to about 90°, preferably about 90°.

4. A Petri dish in accordance with any preceding Claim, wherein the radius of curvature of the curved section is from about 0.5 to about 1.5 percent of the diameter of the bottom wall (15), preferably from about 0.7 to about 0.9 percent of the diameter of the bottom wall.

5. A Petri dish in accordance with any preceding Claim, wherein a projection (27) is provided on the bottom surface of the bottom wall.

6. A Petri dish in accordance with Claim 5 wherein the projection (27) is provided by extending the second straight section (25) beyond the junction of the straight section with the bottom wall to provide a circumferential projection.

7. A Petri dish in accordance with Claim 6 wherein the circumferential projection has an arcuate cross-section.

8. A Petri dish in accordance with any preceding Claim, wherein the height of the container from the top of the side wall to the bottom of the bottom wall is from about 10 to about 15 mm.

9. A Petri dish in accordance with any preceding Claim which has a diameter at the top of the wall of from about 75 to about 100 mm.

10. A Petri dish in accordance with any preceding Claim, wherein the side wall extends from said bottom wall at an angle C from a line normal to the bottom wall of from about 0° to about 5°, preferably about 3°.

11. A Petri dish in accordance with any preceding Claim, wherein the outside cylindrical surface of the side wall is abraded in an area extending upwardly from the junction of the side wall and transition zone.

12. A Petri dish in accordance with any preceding Claim, wherein the lid has a substantially flat top wall (31) merging into a downwardly extending substantially cylindrical side wall (33) having an inner cylindrical surface with a diameter slightly larger than the diameter of the upwardly extending side wall (17) of the container, the top wall merging through a straight slanted section to a depressed area (35) which is radially spaced from the side wall a distance sufficient to receive the projection (27) of a container when a plurality of Petri dishes are stacked.

13. A Petri dish in accordance with Claim 12 wherein the straight slanted section (37) leads from the top wall to the depressed area at an angle D relative to a line normal to the plane of the top wall of from about 10° to about 18°, preferably about 15°.

14. A Petri dish in accordance with Claim 12 or 13 wherein the termination of the inner surface of the side wall (33) has an arcuate projection (43).

15. A Petri dish in accordance with any of Claims 12 to 14 wherein the side wall (33) extends downwardly at an angle E normal to the plane of the top wall (31) of from about 0° to about 5°, preferably about 3°.

**Patentansprüche**

1. Petrischale zum Kultivieren von Mikroorganismen, welche einen Deckel (13) und einen Bodenbehälter (14) umfaßt, der eine im wesentlichen flache Bodenwand (15) aufweist, die in eine aufrechte, im wesentlichen zylindrische Seitenwand (17) über eine Übergangszone eintaucht, die sich zwischen der Bodenwand und der Seitenwand erstreckt, dadurch gekennzeichnet, daß die Übergangszone einschließt:

a) einen ersten geraden Abschnitt (21), der an die Seitenwand angrenzt und sich von der Seitenwand zur Bodenwand hin unter einem Winkel (A) relativ zur Ebene der Bodenwand erstreckt;

b) einen gekrümmten Abschnitt (23), der an den ersten geraden Abschnitt angrenzt; und

c) einen zweiten geraden Abschnitt (25), der an den gekrümmten Abschnitt angrenzt und sich von dem gekrümmten Abschnitt zur Bodenwand unter einem Winkel (B) relativ zur Ebene der Bodenwand erstreckt.

2. Petrischale nach Anspruch 1, bei der der Winkel (A) von etwa 1° bis etwa 5°, vorzugsweise etwa 3° beträgt.

3. Petrischale nach Anspruch 1 oder 2, bei der Winkel (B) von etwa 80° bis etwa 90°, vorzugsweise etwa 90° beträgt.

4. Petrischale in Übereinstimmung mit irgendeinem voranstehenden Anspruch, bei der der Krümmungsradius des gekrümmten Abschnittes von etwa 0,5 bis etwa 1,5% des Durchmessers der Bodenwand (15), vorzugsweise von etwa 0,7 bis 0,9% des Durchmessers der Bodenwand beträgt.

5. Petrischale in Übereinstimmung mit irgeneinem voranstehenden Anspruch, bei der ein vorspringender Bereich (27) an der Bodenfläche der Bodenwand vorgesehen ist.

6. Petrischale in Übereinstimmung mit Anspruch 5, bei der der vorspringende Bereich (27) durch Verlängerung des zweiten geraden Abschnittes (25) über die Verbindung des geraden Abschnittes mit der Bodenwand gebildet ist, um einen umfänglichen vorspringenden Teil vorzusehen.

7. Petrischale in Übereinstimmung mit Anspruch 6, bei der der umfängliche Vorsprung einen gekrümmten Querschnitt aufweist.

8. Petrischale in Übereinstimmung mit irgendeinem voranstehenden Anspruch, bei der die Höhe des Behälters von der Oberseite der Seitenwand bis zum Boden der Bodenwand von etwa 10 bis etwa 15 mm beträgt.

9. Petrischale in Übereinstimmung mit irgendeinem voranstehenden Anspruch, welche einen Durchmesser an der Oberseite der Wand von etwa 75 bis 100 mm aufweist.

10. Petrischale in Übereinstimmung mit irgendeinem voranstehenden Anpsruch, bei der die Seitenwand sich von der Bodenwand unter einem Winkel (C) gegenüber einer Linie, die senkrecht zur Bodenwand verläuft, von etwa 0° bis etwa 5°, vorzugsweise etwa 3° erstreckt.

11. Petrischale in Übereinstimmung mit irgendeinem voranstehenden Anspruch, bei der die äußere zylindrische Oberfläche der Seitenwand in einem Bereich abgeschliffen ist, der sich von der Verbindung der Seitenwand mit der Übergangszone nach oben erstreckt.

12. Petrischale in Übereinstimmung mit irgendeinem voranstehenden Anspruch, bei der der Deckel eine im wesentlichen flache Oberwand (31) aufweist, welche in eine nach unten ragende, im wesentlichen zylindrische Seitenwand (33) eintaucht, welche eine innere zylindrische Fläche mit einem Durchmesser aufweist, der geringfügig größer ist als der Durchmesser der nach oben ragenden Seitenwand (17) des Behälters, wobei die obere Wand durch einen geraden geneigten Abschnitt in einen eingesenkten Bereich (35) eintaucht, der radial im Abstand von der Seitenwand mit einem Abstand angeordnet ist, der ausreicht, den Vorsprung (27) eines Behälters aufzunehmen, wenn mehrere Petrischalen gestapelt sind.

13. Petrischale in Übereinstimmung mit Anspruch 12, bei der der gerade geneigte Abschnitt (37) von der oberen Wand zu dem eingesenkten Bereich unter einem Winkel (D) relativ zu einer Linie senkrecht zur Ebene der oberen Wand führt, wobei der Winkel von etwa 10° bis etwa 18°, vorzugsweise etwa 15° beträgt.

14. Petrischale in Übereinstimmung mit Anspruch 12 oder 13, bei der das Ende der inneren Fläche der Seitenwand (33) einen gekrümmten Vorsprung (43) aufweist.

15. Petrischale in Übereinstimmung mit irgendeinem der Ansprüche 12 bis 14, bei der die Seitenwand (33) sich nach unten unter einem Winkel (E) senkrecht zur Ebene der oberen Wand (31) von etwa 0° bis etwa 5°, vorzugsweise etwa 3° erstreckt.

## Revendications

1. Une boîte de Petri pour la culture de micro-organismes, comprenant:

un couvercle (13) et un récipient de base (11) comportant une paroi de fond (15) sensiblement plane, se raccordant à une paroi latérale sensiblement cylindrique (17), dirigée vers le haut, par l'intermédiaire d'une zone de transition s'étendant entre la paroi de fond et la paroi latérale; caractérisée en ce que la zone de transition comporte:

(a) une première partie droite (21) adjacente à la paroi latérale qui s'étend depuis la paroi latérale en direction de la paroi de fond en faisant avec le plan de la paroi de fond un angle A,

(b) une partie incurvée (23) adjacente à la première partie droite; et

(c) une seconde partie droite (25) adjacente à la partie incurvée qui s'étend de la partie incurvée à la paroi de fond en faisant un angle B avec le plan de la paroi de fond.

2. Une boîte de Petri selon la revendication 1, dans laquelle l'angle A est compris entre environ 1° et environ 5°, en étant de préférence d'environ 3°.

3. Une boîte de Petri selon la revendication 1 ou 2, dans laquelle l'angle B est compris entre environ 80° et environ 90°, en étant de préférence d'environ 90°.

4. Une boîte de Petri selon l'une quelconque des revendications précédentes, dans laquelle le rayon de courbure de la partie incurvée est compris entre environ 0,5 et environ 1,5 pour cent du diamètre de la paroi de fond (15), en étant compris de préférence entre environ 0,7 et environ 0,9 pour cent du diamètre de la paroi de fond.

5. Une boîte de Petri selon l'une quelconque des revendications précédentes, dans laquelle une saillie (27) est formée sur la surface inférieure de la paroi de fond.

6. Une boîte de Petri selon la revendication 5, dans laquelle la saillie (27) est formée en prolongeant la seconde partie droite (25) au-delà de la jonction de la partie droite avec la paroi de fond afin de créer une saillie circonférentielle.

7. Une boîte de Petri selon la revendication 6, dans laquelle la saillie circonférentielle a une section droite incurvée.

8. Une boîte de Petri selon l'une quelconque des revendications précédentes, dans laquelle la hauteur du récipient depuis le sommet de la paroi latérale jusqu'au bas de la paroi de fond est comprise entre environ 10 et environ 15 mm.

9. Une boîte de Petri selon l'une quelconque des revendications précédentes, qui présente au sommet de la paroi un diamètre compris entre environ 75 et environ 100 mm.

10. Une boîte de Petri selon l'une quelconque des revendications précédentes, dans laquelle la paroi latérale s'étend à partir de la paroi de fond en faisant, avec une droite perpendiculaire à la paroi de fond, un angle C compris entre environ 0° et environ 5°, de préférence d'environ 3°.

11. Une boîte de Petri selon l'une quelconque des revendications précédentes, dans laquelle la surface cylindrique externe de la paroi latérale est abrasée dans une zone s'étendant vers le haut à partir de la jonction entre la paroi latérale et la zone de transition.

12. Une boîte de Petri selon l'une quelconque des revendications précédentes, dans laquelle le couvercle comporte une paroi supérieure (31) sensiblement plane se raccordant à une paroi latérale (33) sensiblement cylindrique, s'étendant vers le bas et comportant une surface cylindrique interne ayant un diamètre légèrement plus grand que le diamètre de la paroi latérale (17) s'étendant vers le haut, du récipient, la paroi supérieure se raccordant par l'intermédiaire d'une partie droite inclinée à une zone en creux (35) qui est radialement espacée de la paroi latérale d'une distance suffisante pour recevoir la saillie (27) d'un récipient lorsque plusieurs boîtes de Petri sont empilées.

13. Une boîte de Petri selon la revendication 12, dans laquelle la partie droite inclinée (37) s'étend de la paroi supérieure jusqu'à la zone en creux en faisant, par rapport à une droite perpendiculaire au plan de la paroi supérieure, un angle D compris entre environ 10° et environ 18°, de préférence d'environ 15°.

14. Une boîte de Petri selon la revendication 12 ou 13, dans laquelle la terminaison de la surface interne de la paroi latérale (33) comporte une saillie incurvée (43).

15. Une boîte de Petri selon l'une quelconque des revendications 12 à 14, dans laquelle la paroi latérale (33) s'étend vers le bas en faisant, avec le plan de la paroi supérieure (31), un angle E compris entre environ 0° et environ 5°, de préférence d'environ 3°.

0 124 212

FIG. 1

FIG. 2A

FIG. 2B

0 124 212

FIG. 4

FIG. 3

FIG.8

2

FIG.6

FIG.5A

FIG.7

FIG.5B

FIG. 9A

FIG. 9B

FIG. 9C

FIG. 9D